# EUROPEAN PATENT APPLICATION

(11) **EP 4 252 789 A1**
(43) Date of publication of application: **04.10.2023**
(21) Application number: 22382293.3
(22) Date of filing: 29.03.2022
(51) Int. Cl.: A61L 27/18, A61L 27/52, A61L 33/00, C12N 5/0783

(54) **INVERSE OPAL HYDROGEL AND ITS USE**

(71) Applicant: Consejo Superior De Investigaciones Científicas (CSIC), 28006 Madrid (ES)
(72) Inventor: Guasch Camell, Judit, 08193 Bellaterra (Barcelona) (ES); Ratera Bastardas,, Imma, 08193 Bellaterra (Barcelona) (ES); Veciana Miró, Jaume, 08193 Bellaterra (Barcelona) (ES); Santos Abreu, Roberto Fabião, 08193 Bellaterra (Barcelona) (ES); Pérez del Río, Eduardo, 08193 Bellaterra (Barcelona) (ES); Castellote Borrell, Miquel, 08193 Bellaterra (Barcelona) (ES)
(74) Representative: Pons

(57) **Abstract**

The present invention relates to an inverse opal (IOPAL) hydrogel formed with poly(ethylene)glycol (PEG) covalently bonded to heparin (Hep). The invention also relates to the use of said hydrogel as well as to the procedure, based in the inverted colloidal crystal technique, for preparing the same. Said hydrogel has demonstrated to be very effective and useful in cell culture and more particularly in immunotherapy and in organoid culture.

## Description

The invention relates to an inverse opal (IOPAL) hydrogel formed with poly(ethylene)glycol (PEG) covalently bonded to heparin (Hep). The invention also relates to the use of said hydrogel as well as to the procedure, based in inverted colloidal crystal technique, for preparing the same.

### BACKGROUND ART

Adoptive cell therapy (ACT) is an immunotherapy in which T cells are usually harvested from the blood or tumor tissue of the patient, genetically modified and/or selected in the laboratory, expanded *ex vivo* in large numbers, and reintroduced into the same patient.

These cells are modified *ex vivo* to improve the targeting and killing of cancer cells, like in the cases of engineered T cell receptor (TCR) cells and chimeric antigen receptor T cell (CAR T cell) therapies. Alternatively, such as in the case of endogenous T cell therapy (ETC) or tumor-infiltrating lymphocytes (TIL), the cells are selected due to their specificity among the rest. In any case, the resulting T cells are then capable of specifically recognizing and eliminating malignant cells; with the potential of maintaining their healing properties for years through memory subsets, thus helping the patient immune system to fight cancer recurrence.

T cells present a few subsets, which play specific roles in ACT. There are indications of the synergy occurring between CD4+ and CD8+ T cell populations, which could improve antitumor effectivity compared to a single subset. Although the CD8+ subset displays the main cytotoxic function, CD4+ T cells help directing the immune response and activating cytotoxic lymphocytes. Interestingly, CD4+ T cells have recently proven to induce even higher antitumor responses than CD8+ T cells alone, in some specific cases. CD4+ T cells are also less inclined to exhaustion and are gaining more relevance for the prospects of ACT. Moreover, the CD4+ T cell population is an abundant immune cell population in blood and therefore an easy subset to purify, making it more appealing than others for experimentation.

Even though the potential of this cellular therapy is undoubtful, there are several milestones remaining to be overcome in order to achieve a first line anti-cancer therapy. Notwithstanding, the successful application of ACT will require to address its current issues related with the optimization and clinical translation of these treatments, since achieving an optimal T cell *in vivo* proliferation and persistence, which is also determined by the *ex vivo* activation, proliferation, and differentiation before their reintegration into the patient, is still a major limiting step.

The current gold standard method to activate and expand T cells relies on artificial antigen presenting cells (APCs) consisting of polymeric magnetic beads (such as Dynabeads; Thermo Fisher Scientific, USA) that are coated with stimulating antibodies (usually, anti-CD3 and anti-CD28). Although these beads are easy to manipulate and remove by magnetic separation, they are not able to provide environmental signals naturally present in the native secondary lymphoid organs (SLOs).

SLOs and particularly the lymph nodes (LNs), are responsible for coordinating the immune responses in mammals. LN tissues have a well interconnected structure that enhances cellular motility and T cell-APC interactions.

Our bodies have hundreds of scattered LNs, all connected by the lymphatic vessels. These organs, which are mostly bean shaped and range in size from 0.1 to 2.5 cm long, act as a filter that cleanse the lymph from pathogens and cancerous cells. Internally, they are formed by a dense fibrous capsule that contains trabeculae, which are connective tissue strands that extend inward and divide the nodes into various micro-sized compartments. These compartments are indispensable for the function of the LNs, and are the T cell zones and B follicles, where T and B cells reside, respectively.

The inner part or stroma of the LNs consists of reticular fibers, which is a network of extracellular matrix (ECM) proteins, mainly containing collagen type III, but also collagen types I and IV, laminin, fibronectin, entactin, or heparan sulfate, which are the responsible to generate and maintain the micro-compartments.

The LNs are complex organs, and two-dimensional (2D) culture methods are not suitable to mimic the natural three-dimensional (3D) environment of cells *in vivo.* To tackle this limitation, 3D natural or synthetic structures such as hydrogels are used to better mimic the ECM with the objective to recreate the natural environment of cells in a more realistic way than the standard cell culture techniques. Furthermore, hydrogels can also be used as platforms to promote the interaction of cells with different growth factors and proteins, in order to influence different cellular responses, such as differentiation or growth (Raic, A., et al. Biomaterials 35, 929-940 (2014); Caliari, S. R. & Burdick, J. A. Nat. Methods 13, 405-414 (2016)).

Hydrogels consisting of PEG and heparin have been described. More precisely, when dissolved Mal-LMWH (low molecular heparin functionalized with maleimide) and PEG-SH (4-arm thiolated PEG) solutions were mixed together, hydrogels were formed (Baldwin, A. D. et al. J. Biomed. Mater. Res. A 100, 2106-2118 (2012); Baldwin, A. D. & Kiick, K. L. Polym. Chem. 4, 133-143 (2013)).

Poly(ethylene glycol) (PEG) is one of the most studied synthetic materials for cell culture purposes due to its advantageous properties. It is biocompatible, transparent, hydrophilic, and a biologically inert polymer, which prevents unspecific protein attachment. Its physicochemical properties can easily be tuned. For example, its elasticity can be directly modified by the monomer size and polymer concentration during the synthesis. These PEG hydrogels can be produced with high reproducibility under cytocompatibility conditions, as well as functionalized with biomolecules of interest, such as cell adhesive motifs.

Heparin is a sulphated glycosaminoglycan naturally present in the ECM, with a high number of negative charges provided by carboxyl and sulphonyl groups. Although this molecule is commonly known as an anticoagulant and widely used in drug and growth factor delivery systems, it allows the interaction with cationic molecules and basic peptides through its electronegative charge, leading to the mediation of many biological processes.

Furthermore, hydrogels with enlarged and homogeneous pore structures using the inverse opal (IOPAL) may increase the infiltration capacity of cells and their mobility through their structure.

Hydrogels comprising PEG and heparin have been previously produced for cell-support applications. In some cases, (Stachowiak A. N. & Irvine D. J. J. Biomed. Mater. Res. A 85(3), 815-828 (2008)) linear PEG polymers difunctionalized with acrylate groups have been used to produce PEG-containing hydrogels. This approach of using linear groups is known to produce defects in the form of primary loops during the cross-linking of the hydrogel, which results in impaired mechanical properties. Moreover, in this context, the addition of heparin to said hydrogels is performed by subsequent biotin-streptavidin functionalization.

Other efforts have been directed into preparing hydrogels with PEG and heparin in its backbone. In "Niea T. et al. Acta Biomater. 5(3), 865-875 (2009)", a PEG-heparin hydrogel was used to study differences in the response of human aortic adventitial fibroblasts (AoAF) in 2D. Moreover, similar hydrogels have been used in 3D cell experiments. In "Perez del Río E. et al. Biomaterials 259, 120313 ((2020))", 3D PEG-heparin hydrogels were shown to improve cell proliferation when compared to cells growing in suspension. However, both these hydrogels fail to provide an accurate control of the inner structure of the hydrogel (i.e. of its porosity). This control is key for 3D cell experiments, and especially for mimicking organs such as the lymph nodes in which its inner compartmentalized structure is indispensable for their function (Mueller, S. N. & Germain, R. N. Nat. Rev. Immunol. 9, 618 (2009)).

Organoids are 3D multi-cellular structures cultured in vitro which recapitulate multiple aspects of an organ. They are a very useful preclinical model for studying diseases and its treatments. In most cases, organoids need to be supported by a scaffold that mimics the ECM, which is its native environment. Nowadays, most of the state-of-the-art organoid studies use Matrigel^{®}, or to a lower extent, Cultrex^{®} Basement Membrane Extract (BME) as scaffold. Matrigel^{®} and Cultrex^{®} BME are collagen-rich substrates based on solubilized basement membrane matrix secreted by Engelbroth-Holm-Swam (EHS) mouse sarcoma cells and is widely used for cell culture in Cell Biology. However, these matrices have some long-standing drawbacks such as the need to work on ice (Matrigel^{®} jellifies at RT); a high cost and occasional supply shortages; and the difficult working conditions it imposes, denying, for example, the possibility to work with the standard 96-well plates. More importantly, the murine-origin of such matrices has created translational problems due to differences with the human species. In this context, new scaffolds that can support organoid formation and culture would be welcome by cell biologists working on the field of organoids. However, organoids of different cell types demand very specific environments to proliferate, and often need specific cues in the matrix where they are embedded to form and proliferate.

In the present invention, 3D IOPAL hydrogels consisting of PEG and heparin are disclosed. They have been synthesized and characterized to imitate the conditions of the ECM of the LNs, with the objective to increase primary human (CD4+) T cell proliferation and tune differentiation for ACT. CD4+ T cell proliferation was found to be enhanced when using the IOPAL 3D PEG-Hep hydrogels, thus improving not only the state-of-the-art suspension systems, but also the performance of the hydrogel in its bulk form. Moreover, the capacity of such hydrogels to influence the phenotype obtained, naive (T_{N}), effector (T_{EFF}), central memory (T_{CM}), and effector memory (T_{EM}), which is closely related with the clinical outcomes, has been demonstrated. In addition, the hydrogels of the present invention have been found to support tumor organoid formation for various cancer types, improving in some cases the performance of the state-of-the-art 3D systems.

### SUMMARY OF THE INVENTION

In this invention, artificial lymph nodes composed of inverse opal (IOPAL) three-dimensional (3D) poly(ethylene) glycol (PEG) hydrogels covalently combined with heparin are engineered to resemble the environment of the lymph nodes, where T cells get activated and proliferate. The introduction of an IOPAL strategy allowed a precise control on the porosity of the hydrogels, providing an increase in the proliferation of primary human CD4+ T cells when compared with state-of-the-art expansion systems consisting of artificial antigen presenting cells in suspension, and even superior expansion than a hydrogel with the same composition, but without an IOPAL structure. It has been shown the beneficial effect of having an IOPAL architecture in the hydrogels, which helps achieving large numbers of cells while maintaining the desired selected phenotypes required for adoptive cell therapy (ACT), by better mimicking the lymph nodes nature.

The IOPAL hydrogels of the present invention were also found to be surprisingly useful for cancer organoid (tumoroid) formation. While the complexity and specificity of organoid culture suggested that a more elaborated and tumor-specific structure would be needed, IOPAL 3D PEG-heparin hydrogels showed surprisingly good results in organoid culture, both in solid tumors and hematological malignancies. The performance of IOPAL hydrogels improved the state-of-the-art commercial Matrigel^{®} scaffolds in terms of organoid formation, ease of handling and other experimental improvements.

A first aspect of the present invention related to a hydrogel, hereinafter the hydrogel of the invention, comprising a functionalized PEG multi-arm star polymer covalently combined with heparin in the form of inverse opal scaffold.

As it is known in the state of the art, "opal" refers to the crystalline array of close-packed spheres and "inverse" refers to the fact that this array is the negative space, or the resulting pores. Then, an inverse opal structure, or inverted colloidal crystal, is the negative replica of the opal structure, in which the solid spheres are replaced by air phase forming pores whereas the space between spheres is filled with a new material (the hydrogel material in the present invention). Consequently, an inverse opal structure is a porous structure that possess an ordered array of uniform pores. In this invention, the pores have dimensions on the micrometre scale.

These structures are obtained by the inverse opal technique, also called inverted colloidal crystal technique. As known, this technique is based on the use of a colloidal crystal template to subsequently create homogenous (micro)pores in the material upon the elimination of the template. The technique comprises the next steps:
- adding a solution of spheres into a template,
- evaporating the solvent comprising the spheres,
- adding a solution comprising the hydrogel components into the template,
- dissolving the spheres in a solution.

As used herein, the term "hydrogel" refers to a 3D network of a polymeric material which exhibits the ability to swell in water and to retain a significant portion of water within its structure without dissolving.

The terms "poly(ethylene glycol)", "polyethyleneglycol" or "PEG" or "polyethylene oxide" or "polyoxyethylene" are considered equivalents and can be used interchangeably herein.

PEG is a hydrophilic and biocompatible polymer. It offers the possibility to design scaffolds with very well-controlled structural and mechanical properties. The Young's modulus of PEG hydrogels, which is a measure of the mechanical properties, can be tuned 4 orders of magnitude covering the values of all tissues in the human body.

The term "multi-arm star" used herein refers to star-shaped polymers, which are the simplest class of branched polymers with a general structure consisting of at least three linear chains connected to a central core, wherein said core or the centre of the polymer can be an atom, molecule, or macromolecule and the chains, or "arms", consist of variable-length organic chains. Star-shaped polymers in which the arms are all equivalent in length and structure are considered homogeneous, whereas the ones with variable lengths and structures are considered heterogeneous.

In the present invention, the use of PEG multi-arm star polymer is essential to form the hydrogel, otherwise a hydrogel in the form of inverse opal scaffold cannot be formed adequately to be useful in T cell expansion, replication and proliferation, fabrication of artificial LNs and producing large amounts of T cells with therapeutic phenotypes as well as in organoid formation and culture. A linear PEG polymer does give rise to the desired IOPAL hydrogel.

Preferably, the PEG multi-arm star polymer is functionalized with reactive end groups including, without limitation, N-hydroxysuccinimide ester, thiol, carboxyl, carbonyl, primary amine, aldehyde, anhydride, epoxide, maleimide, pyridyl disulfide, amines, hydrazides, isocyanate, sulfonyl chloride, fluorobenzene, imidoester, haloacetyl, vinylsulfone, carbodiimide, alkoxyamines, or iodoacetyl.

More preferably, the PEG-multi-arm polymer in the hydrogel of the invention is functionalized with thiol groups forming thiolated PEG or methoxy PEG thiol or methoxypolyethylene glycol thiol or mPEG thiol, wherein these terms are considered equivalents and can be used interchangeably herein (represented as PEG-SH).

In another preferred embodiment, the functionalized PEG multi-arm star polymer is a functionalized PEG four-arm star polymer; and more preferably is a thiol-functionalized PEG four-arm star polymer of formula:

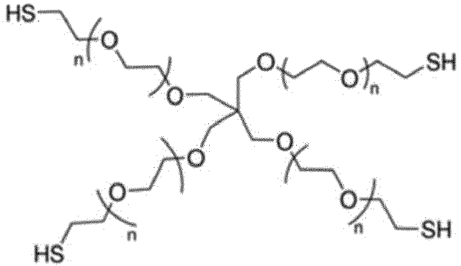

The concentration of the functionalized PEG multi-arm star polymer in the hydrogel of the invention comprising a certain range. In a preferred embodiment of the invention, the concentration of the functionalized PEG multi-arm star polymer in the hydrogel of the invention ranges between 0.5%wt to 6%wt on the total weight percentage of hydrogel.

The terms "% by weight" or "% wt" or "%w/w" are considered equivalents and can be used interchangeably herein and refer to the weight percentage.

In another preferred embodiment of the hydrogel of the invention, the functionalized PEG multi-arm star polymer concentration is between 1.2%wt to 4%wt on the total weight percentage of hydrogel, and even more preferably, is 3%wt.

The concentration of PEG multi-arm star polymer is a key parameter to obtain good hydrogels. PEG weight percentages above 6%wt were found to give solubility problems, and thus are not suitable for hydrogel formation. When the concentration of PEG is too low, for example below 0.5%wt, or even below 1.2%wt, the cross-linking is too weak and the mixture results in a viscous fluid rather than in a hydrogel.

Further, the hydrogel of the invention comprises heparin. Heparin is a naturally occurring highly sulphated glycosaminoglycan (GAG). Therefore, it has combined advantages such as being a biomolecule present in the human body, presenting a very negative charge that allows for electrostatic interactions with molecules of interest, and having the possibility to functionalize it with functional groups such as maleimide for different applications, like supramolecular chemistry or materials science.

A non-fractionated heparin is preferably used in the present invention. Non-fractionated heparin, also referred as unfractionated heparin or UFH, refers to a type of heparin that has not been treated or subjected to purification to obtain low molecular weight heparin.

The advantages of using non-fractionated heparin over further processed heparins comprise a lower cost and the ease to have a steady supply, both things derived from the simpler production process.

In another more preferred embodiment, the hydrogel of the invention comprises functionalized heparin, more preferably, maleimide-functionalized heparin, hereinafter Hep-Mal.

The use of Hep-Mal allows to form hydrogels through the reaction of maleimide with thiols that can be incorporated to the PEG molecules.

The maleimide-thiol reaction, which is a Michael reaction, offers many advantages versus other types of reactions used for hydrogel formation, such as the well-known acrylate cross-linking which requires UV light. First, the maleimide-thiol reaction is a "Click Chemistry" strategy, which means that is fast, results in a high yield, and happens in mild and biocompatible conditions. Secondly, it does not require UV light, which is harmful for cell viability as it introduces DNA damage. Finally, maleimide offers a very versatile anchoring point for biological molecules, as it can be crosslinked with available thiol groups through Cys amino acids of proteins.

The functionalized PEG multi-arm star polymer is covalently combined with Hep-Mal forming a PEG-Hep hydrogel. The PEG-Hep hydrogel is formed through a reaction between the maleimide of the functionalized heparin and the reactive end groups of the functionalized PEG-multi-arm, that result in a covalent crosslink and the consequent gelation. The reactive end group of the functionalized PEG multi-arm have been previously described herein and apply equally to this embodiment.

In a preferred embodiment of the invention, the concentration of heparin, or of functionalized heparin in case it is functionalized, in the hydrogel of the invention ranges between 1%wt to 10%wt on the total weight percentage of hydrogel.

In another preferred embodiment of the hydrogel of the invention, the concentration of heparin, or of functionalized heparin in case it is functionalized, is between 2%wt to 6%wt on the total weight percentage of hydrogel, and even more preferably, is 4.5%wt.

In another preferred embodiment of the hydrogel of the invention, the reactive end group of the functionalized PEG multi-arm is a thiol, primary amine or an epoxide group, more preferably a thiol group. So, the PEG-Hep hydrogel is formed through a maleimide-thiol covalent reaction between the maleimide of the functionalized heparin and the thiols of the functionalized PEG multi-arm.

In an embodiment, the hydrogel of the invention comprises at least one positively charged immune molecule, such as cytokines, cell adhesion molecules or growth factors. But in a preferred embodiment, the hydrogel does not comprise positively charged immune molecules. In particular, it does not comprise cytokines, cell adhesion molecules or growth factors.

Preferably, the hydrogel consists of water and functionalized PEG multi-arm star polymer covalently combined with heparin. More preferably, the hydrogel consists of water and thiol-functionalized PEG multi-arm star polymer covalently combined with maleimide-functionalized heparin. Even more preferably, the hydrogel consists of water and a thiol-functionalized PEG 4-arm star polymer covalently combined with maleimide-functionalized non-fractionated heparin.

The term "positively charged immune molecule" used herein, refers to molecules, particularly proteins, that are involved in mounting the immune response, especially the adaptive immune response associated with lymphocyte activation, and in particular T cell activation, which results in intracellular signalling events and inflammation, and have a positive net electrical charge for anchoring with the hydrogel of the invention. Examples of positively charged immune molecules include, without limitation, cytokines, cell adhesion molecules and growth factors.

The term "cytokine" used herein, refers to small proteins or glycoproteins having a molecular mass of less than about 30 kDa, that are produced by cells of the immune system and that regulate or modulate inflammation, cancer, or immune responses, such as modulation of the effector function of T cells, B cells, natural killers (NK) cells macrophages, APC, or other immune system cells.

The term "cell adhesion molecule" used herein, refers to molecules, particularly proteins, located on the cell surface involved in binding with other cells or with the ECM in the cell adhesion process affecting cellular mechanisms including, without limitation, growth, migration, proliferation, contact inhibition, differentiation, or apoptosis. Examples of cell adhesion molecules include, without limitation, integrin ligands such as ECM proteins like fibronectin, vitronectin, laminin, collagen, intercellular adhesion molecules (ICAMs), vascular cell adhesion molecule (VCAM), cadherins, or selectins.

In a preferred embodiment, the hydrogel of the present invention has an average pore between 10 and 200 µm, preferably between 47 and 165 µm, more preferably between 70 and 100 µm, even more preferably between 75 and 80 µm, as measured by environmental scanning electron microscopy (ESEM).

The pore architecture of the hydrogel is a key feature for its performance. For cells to have enough space to grow and interact with each other, a certain pore size must be ensured. The lymph node is the organ in the human body where T cells expand. Therefore, the porosity of collagen fibres in the LNs, which is around 80 µm, is expected to be optimal and result in unconstrained migration.

Together with the pore size, the expansion of cells inside a biomaterial will strongly depend on the capability of cells to migrate within the material. Therefore, pores should be well connected with each other. A connectivity density of at least 5000 cm⁻³ is preferred; a connectivity above 10000 cm⁻³ or even above 18000 cm⁻³ is preferred for an optimal cell expansion, measured by X-ray microtomography.

The term "connectivity density" used herein, refers to the connectivity number divided by the volume of interest (i.e. the analysed volume of sample) as obtained from the analysis of X-ray microtomography measurements using a Skyscan 1272 high-resolution micro computed tomography (Bruker, USA). Connectivity is a basic concept in mathematics and computer science which equals the minimum number of elements which need to be removed to disconnect the remaining nodes from each other. Connectivity has no units, and it relates to the degree to which parts of an object are multiply connected. Connectivity can be thought of as how many possible ways exist to travel through the pores of the material.

The IOPAL hydrogels of the invention have the advantage of possessing a narrow distribution of pore sizes, a strong control over the pore average size, and a high connectivity density. These features make the IOPAL hydrogels of the invention very well suited for expanding cells, especially T cells, and cultivating organoids, especially tumoroids of solid cancers.

In a preferred embodiment, the hydrogel of the present invention has a storage shear modulus (G') between 0.05 and 10 kPa, preferably between 0.1 and 5 kPa, more preferably between 0.4 and 2 kPa, as measured by small-amplitude oscillatory shear (SAOS) rheology.

Another aspect of the present invention refers to an intermediate product that is obtained when preparing the hydrogel of the present invention. This intermediate product is a composition comprising the hydrogel as defined in the first aspect of the invention and non-crosslinked polymeric spheres.

The term "non-crosslinked polymeric spheres" refers to spheres made of a polymer that has not been subjected to any cross-linking, as opposed to crosslinked polymeric spheres, which are widely used in different fields within the materials science field.

In a preferred embodiment of this aspect of the invention, the spheres are PMMA (polymethacrylate) or polystyrene non-crosslinked spheres, more preferably, the spheres are PMMA non-crosslinked spheres.

In a preferred embodiment of this aspect of the invention, the spheres have a diameter between 10 and 200 µm, preferably between 47 and 165 µm, more preferably between 70 and 100 µm, even more preferably between 75 and 80 µm.

Another aspect of the invention refers to the method for preparing the hydrogel described in the first aspect of the invention. The method is based in the inverted colloidal crystal technique and comprises the following steps:
i. adding a suspension of non-crosslinked polymeric spheres into a template,
ii. evaporating the solvent comprising the non-crosslinked spheres,
iii. adding an aqueous solution comprising the hydrogel components (i.e: functionalized PEG multi-arm star polymer and functionalized heparin) into the template and maintain the mixture during a time of at least 24 hours to allow the hydrogel components to react with each other and to jellify,
iv. dissolving the non-crosslinked polymeric spheres by adding a solution comprising an acid or an organic solvent to the mixture obtained in step iii).

In a preferred embodiment of the process of the present invention, the suspension of step i. is an aqueous suspension.

In a preferred embodiment, the spheres are PMMA (polymethacrylate) or polystyrene non-crosslinked spheres, more preferably, the spheres are PMMA non-crosslinked spheres.

In a preferred embodiment of this aspect of the invention, the spheres have a diameter between 10 and 200 µm, preferably between 47 and 165 µm, more preferably between 70 and 100 µm, even more preferably between 75 and 80 µm.

In a preferred embodiment, the evaporation step ii) is carried out at a temperature not higher than 40 ºC, preferably between 15 and 30 ºC, even preferably between 20 and 25 ºC.

In a preferred embodiment of the process of the present invention, the mixture is maintained in step iii) at a temperature between 20 and 50 ºC, preferably between 30 and 40 ºC, even preferably at 37 ºC.

In a preferred embodiment of the process of the present invention, the solution of step iv. comprises an acid, preferably a carboxylic acid, more preferably acetic acid.

In a preferred embodiment, the duration of step iv. is between 1 and 5 days, preferably between 2 and 4 days, more preferably about 3 days.

In a preferred embodiment of the process of the present invention, no annealing step is used. Usually, an annealing step (mainly thermal annealing) is carried out in the processes of the state of the art after step i) (between i and ii) in order to fuse together the polymeric particles. However, in the present invention, no annealing step is used. This fact provides the advantage of simplifying the process by avoiding one step and the requirement of having an oven. Moreover, progressive and slow evaporation of the solvent drives particles together to create an ordered and closed-packed array that results in homogenous well-connected pores.

In another aspect, the present invention relates to a hydrogel in the form of an inverse opal scaffold obtained by the process described above.

As explained previously at the present description, the inventors have developed an alternative scaffold that comprises a PEG-Hep hydrogel mimicking the ECM of LNs for T cell expansion, replication and proliferation, fabrication of artificial LNs and producing large amounts of T cells with therapeutic phenotypes.

Then, another aspect of the invention is the hydrogel of the invention or the composition of the invention for use in immunotherapy, preferably in cancer treatments, autoimmune diseases and/or infections.

The term "treating" (or "treat" or "treatment") used herein refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders associated with immune diseases, cancer or infections.

The terms "immunotherapy" used herein, refer to the set of treatment strategies to stimulate, inhibit, or replenish the immune system against cancer, infections, or other diseases as well as to lessen the side effects of very aggressive treatments used against cancer.

Another preferred embodiment is the hydrogel of the invention or the composition of the invention for use in T cell based adoptive immunotherapy.

The terms "T cell based adoptive immunotherapy", "adoptive cell transfer", "adoptive cell therapy", "adoptive T cell therapy", or "ACT" are considered equivalents and can be used interchangeably herein, refer to a therapeutic approach which involves the isolation and reinfusion or transfer of T lymphocyte immune cells into patients to treat disease, preferably to treat cancer, autoimmune disease, or infections. Types of adoptive cell therapy include chimeric antigen receptor T cell (CAR T cell) therapy, T cell receptor (TCR) gene-modified T cell therapy, tumor-infiltrating lymphocyte (TIL) therapy, lymphokine-activated killer (LAK) cell therapy, and cytokine-induced killer (CIK) cell therapy. The cells may have originated from the patient or from another individual. In autologous cancer immunotherapy, cells are extracted from the patient, cultured in vitro and returned to the same patient. Comparatively, allogeneic therapies involve cells isolated and expanded from a donor separate from the patient receiving the cells.

Examples of types of cancer suitable for immunotherapy include, without limitation, melanoma, colorectal carcinoma, cervical cancer, lymphoma, leukaemia, bile duct cancer, neuroblastoma, lung cancer, breast cancer, or sarcoma.

Cellular immunotherapies require cell culture, and activation, expansion, proliferation, and differentiation of the immune cells, preferably of the T cells. The higher immune cell proliferation rates are relevant for producing the necessary quantities of therapeutic cells to reach the adequate clinical doses. As described previously, by the hydrogel of the invention, the inventors obtained high proliferation parameters and produced large amounts of T cells.

Thus, another aspect of the invention refers to the use of the hydrogel of the invention for *in vitro* cell culture, preferably for 3D human cell culture, more preferably for immune cell culture, and even more preferably for T cell culture.

In another more preferred embodiment is the use of the hydrogel of the invention or the composition of the invention for *in vitro* activation, expansion, proliferation, and/or differentiation of T cells.

The term "T cell activation" used herein refers to a process which T cells identify small numbers of specific foreign antigens, usually peptides, through receptors at the surface and lead to proliferation and differentiation of T cells.

The terms "T cell expansion" or "T cell replication" used herein refer to a process or response of expanding, widening or extending the T cell populations which correlates with the proliferative process. The expansion and replication parameters correlate with a high quantity of cells after the proliferative process.

The term "T cell proliferation" used herein refers to the cell division and cell growth of the cells from the original population to increase the number of T cells.

The term "T cell differentiation" used herein refers to the process in which a T cell changes from one T cell phenotype to another more specialized or mature. Examples of T cell differentiation states include, without limitation, naive T cells, central memory T cells, effector memory T cells, or effector T cell.

The T cells originate as precursor cells, derived from bone marrow, and develop into several distinct types of T cells once they have migrated to the thymus gland. T cell differentiation continues even after they have left the thymus. T cells are grouped into various subsets based on their function.

Types of T cells include, without limitation, helper CD4+ T cells, cytotoxic CD8+ T cells, regulatory T cells, natural killer T cells (NK T cells), and gamma delta T cells (γδ T cells).

The "T helper cells", "Th cells", or "CD4+ T cells" terms used interchangeably herein, refer to a type of T cell that expresses the surface protein CD4 and plays an important role in the immune system, particularly in the adaptive immune system. They help the activity of other immune cells to suppress or regulate immune responses to pathogens and tumor cells. This anti-tumor activity of the CD4+ T cell is used in many immunotherapy techniques, like adoptive cellular therapy (ACT), where autologous cancer-reactive T cells are reinfused into the patient after an ex vivo treatment for tumor destruction.

Consequently, another more preferred embodiment is the use of the hydrogel of the invention or the composition of the invention for CD4+ T cell culture.

In another even more preferred embodiment, there is the use of the hydrogel of the invention or the composition of the invention for activation, expansion, proliferation, and/or differentiation of CD4+ T cells.

Another aspect of the invention refers to the use of the hydrogel of the invention for *in vitro* organoid culture.

The term "organoid" refers to a 3D multicellular *in vitro* tissue construct that mimics its corresponding *in vivo* organ. Therefore, it morphologically and/or functionally resembles an organ structure in such a way that it can be used to study aspects of that organ.

Very surprisingly, even if not specifically designed for that purpose, the IOPAL hydrogel of the invention has shown incredibly good capability for supporting the formation of cancer organoids or tumoroids of both solid tumors and hematological malignancies. The hydrogels of the invention are easy to work with at room temperature conditions and in standard 96-well plates, which overcomes some of the long-standing problems in the field of organoids culture.

In a preferred embodiment, the hydrogel of the invention for use in organoid culture comprises an ECM protein or cell binding sequence, preferably an ECM protein selected from ICAM-1 and VCAM-1 or a cell binding sequence selected from RGD, LDV, and GFHypGER.

| Cell binding sequence abbreviation | Amino acid sequence |
|---|---|
| RGD | Arg-Gly-Asp |
| LDV | Leu-Asp-Val |
| GFHypGER (SEQ ID NO: 1) | Gly-Phe-hydroxyproline-Gly-Glu-Arg |

In summary, the IOPAL hydrogel described in the present invention provides an improved performance with respect to the hydrogels of the state of the art. Some of the advantages of the hydrogel of the present invention are indicated below:
- The use of a multi-arm PEG provides an important advantage to the hydrogel of the invention. It eliminates the defects that arise from linear structures and results in a robust gelling process.
- The click chemistry of thiol-maleimide exploited in the design of the hydrogel of the invention is fast and does not give any side product, while other cross-linking strategies demand harsher conditions, such as UV radiation. Moreover, this strategy allows to easily modify the hydrogel composition by using biomolecules functionalized with either thiol or maleimide.
- The IOPAL design gives the hydrogel of the invention a controlled and homogenous pore size. This pore size can be easily tailored to match that of the ECM of the lymph nodes or other healthy or cancerous organs in order to better suit specific cell types.

Moreover, the excellent connectivity between pores may be tuned to result in unconstrained migration of cells within the material.
- Improved (CD4+) T cell proliferation and differentiation. The hydrogel of the invention enhances the expansion of (CD4+) T cells compared to the bulk hydrogel form and promotes the differentiation into clinically relevant T cell subtypes.
- In terms of supporting organoid culture, the hydrogel of the invention is cheaper and easier to manipulate than the state-of-the-art commercial materials.
- Most surprisingly, the hydrogel of the invention supported organoid culture successfully even without any specific recognition domain for the tested organoid types.
- The hydrogel of the invention does not have murine origin as the commercial products, and it can be tuned to mimic the conditions of human biology, thus reducing incompatibilities between species. Consequently, the hydrogel of the invention could resemble better clinical testing, minimizing translation problems.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### DESCRIPTION OF THE DRAWINGS

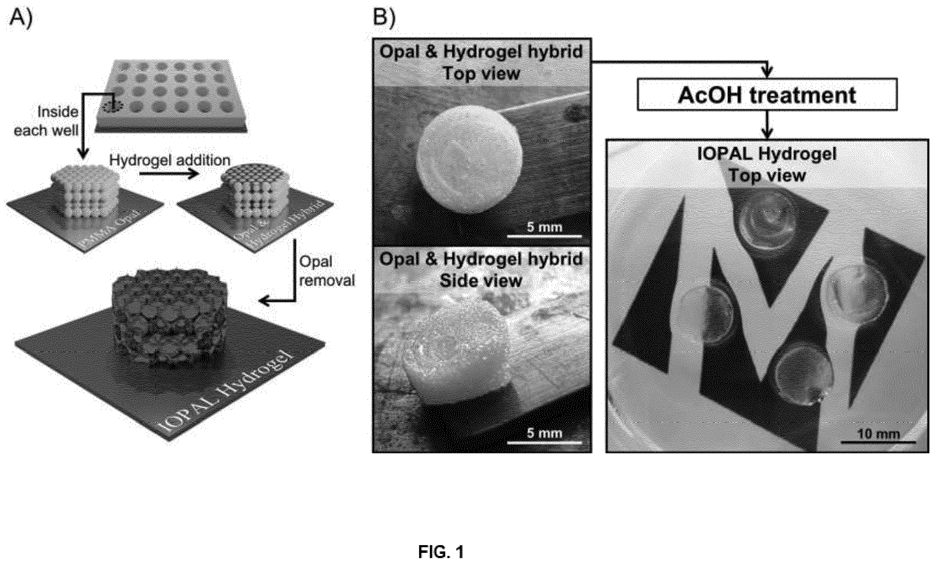

**Fig. 1****.** Formation of the IOPAL PEG-Hep hydrogel. A) Simplified scheme of the formation of an IOPAL PEG-Hep hydrogel. B) Photographs of the opal & hydrogel hybrid before the acetic acid (AcOH) treatment, where they are very white and opaque. After AcOH treatment, they result in transparent hydrogels, indicating the complete removal of the PMMA spheres.

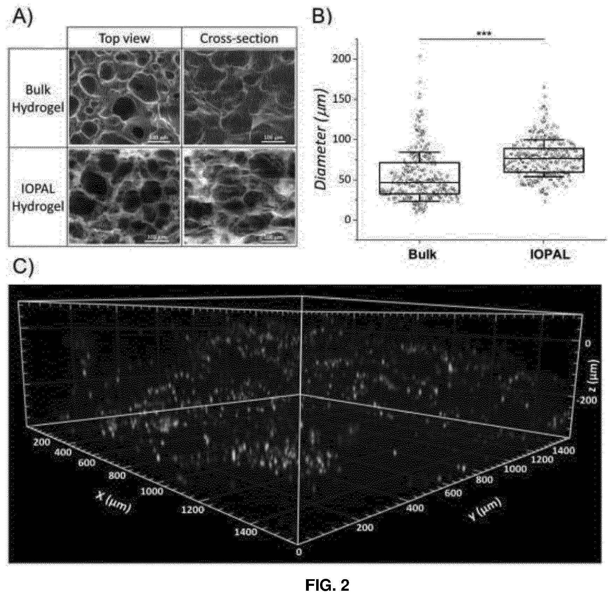

**Fig. 2****.** Structural properties of IOPAL PEG-Hep hydrogels. A) SEM images and B) pore size evaluation of bulk and IOPAL PEG-Hep hydrogels. The statistical significance was determined by the Kruskal Wallis ANOVA test (***p < 0.001). C) 3D confocal projection showing CFSE-stained primary human CD4+ T cells in a representative IOPAL PEG-Hep hydrogel after 5 days of incubation (area = 1.5 cm x 1.5 cm x 0.4 cm).

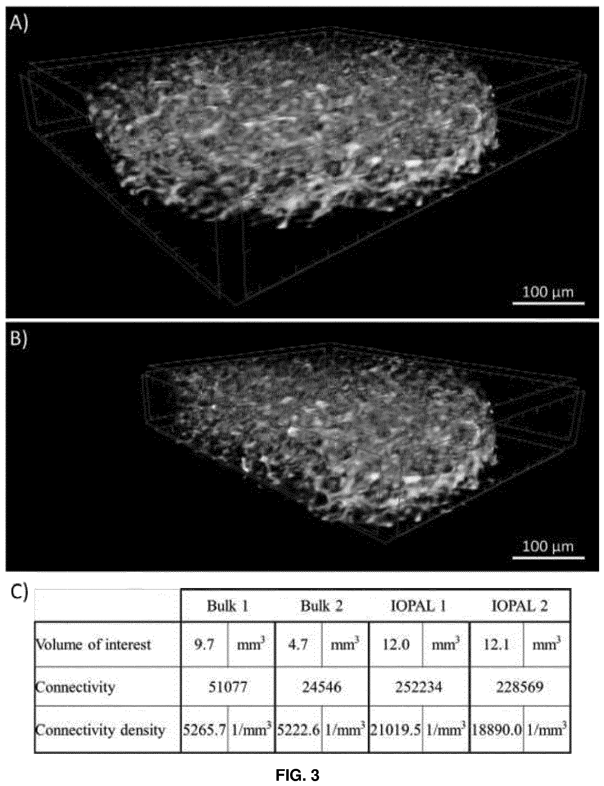

**Fig. 3****.** X-ray tomography analysis of PEG-Hep hydrogels. A) X-ray microtomographs of a representative volume of interest and its B) cross-section of an IOPAL PEG-Hep hydrogel of 1 cm of diameter used to analyze its connectivity. C) Connectivity density obtained (connectivity/volume of interest) for bulk and IOPAL PEG-Hep hydrogels (N_{hydrogels} = 2).

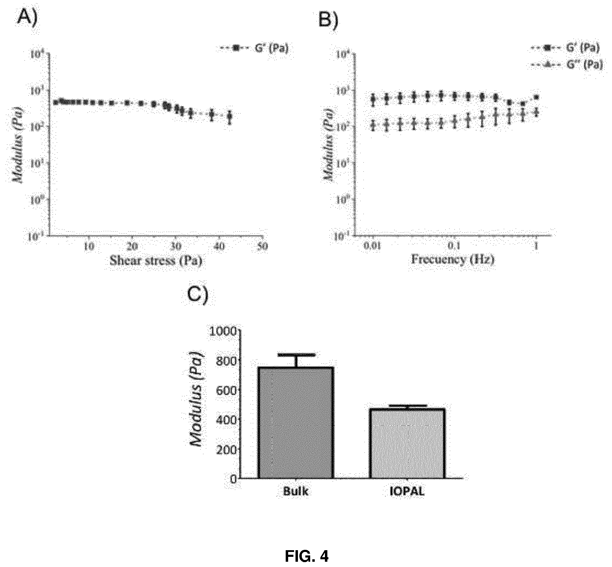

**Fig. 4****.** Small-amplitude oscillatory shear (SAOS) rheology. A) Strain sweeps and B) frequency sweeps of IOPAL PEG-Hep hydrogels (N_{Hydrogels} = 2). C) Storage moduli G' of the IOPAL (0.46 ± 0.01 KPa) and bulk (0.75 ± 0.08 KPa) hydrogels.

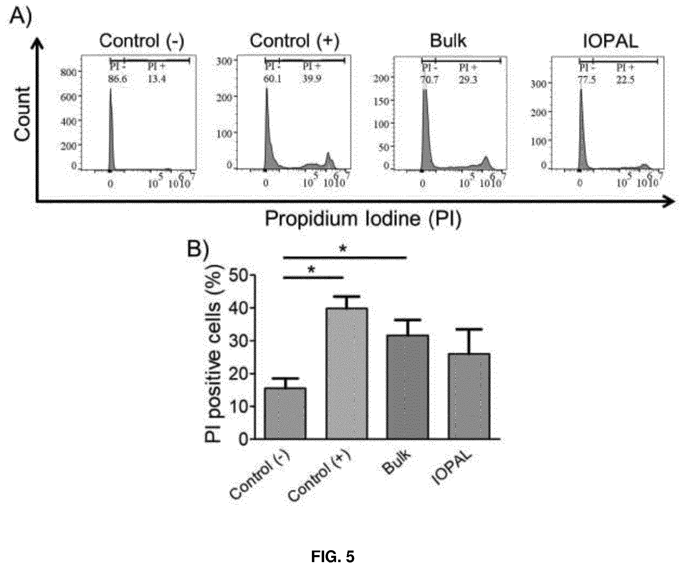

**Fig. 5****.** Effect of IOPAL and bulk PEG-Hep hydrogels on CD4+ T cell viability. A) Representative flow cytometry histograms of the B) propidium iodide (PI) viability test performed to CD4+ T cells seeded for 5 days with Dynabeads on IOPAL and bulk PEG-Hep hydrogels, or in suspension (Control (+)). Control (-) represents cells in suspension with no Dynabeads. Bars are mean + standard deviation (N_{donors} = 4). Significance was determined by the Mann-Whitney U test (*p < 0.05).

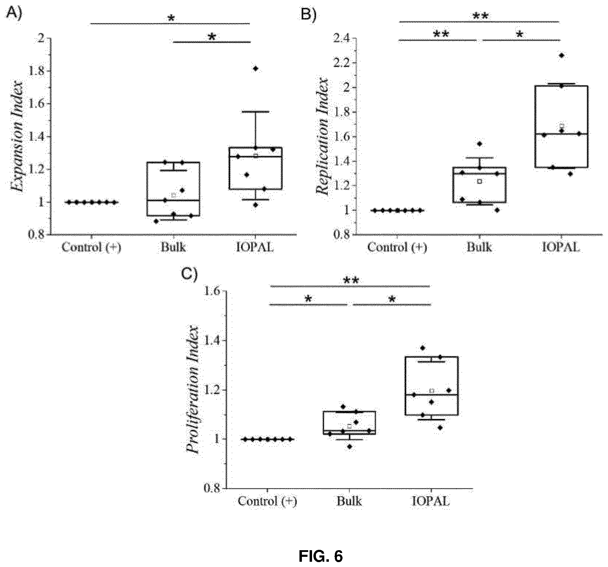

**Fig. 6****.** Effect of PEG-Hep hydrogels on its bulk and IOPAL form on CD4+ T cell proliferation. A) Normalized expansion, B) replication, and C) proliferation indexes of CD4+ T cells stimulated with Dynabeads 6 days after seeding on bulk and IOPAL PEG-Hep hydrogels (N_{donors} = 7). Statistical significance was determined by the Mann-Whitney U test (*p < 0.05, **p < 0.01).

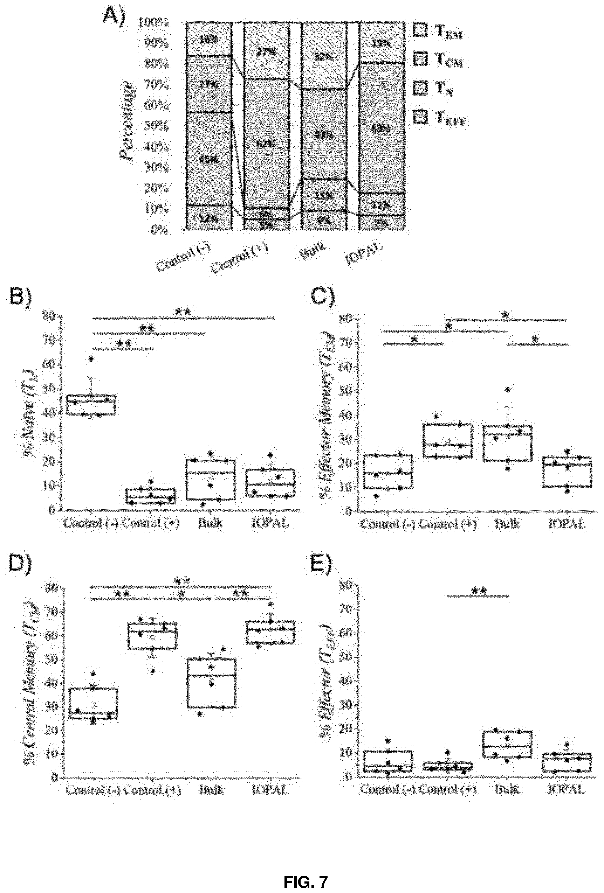

**Fig. 7****.** A) Percentage of naive (T_{N}), effector memory (T_{EM}), effector (T_{EFF}), and central memory (T_{CM}), CD4+ T cells on day 5 (N_{donors} = 6). Box plot representations of the data summarized in A) for the B) naive (T_{N}), C) effector memory (T_{EM}), D) central memory (T_{CM}), and E) effector (T_{EFF}) CD4+ T cells showing the results of the Mann-Whitney U tests (* p < 0.05 and ** p < 0.01) performed. The negative control consists of cells seeded in suspension without Dynabeads, whereas in the positive control, cells are seeded with Dynabeads. When cells are seeded in the hydrogels, they are always stimulated with Dynabeads.

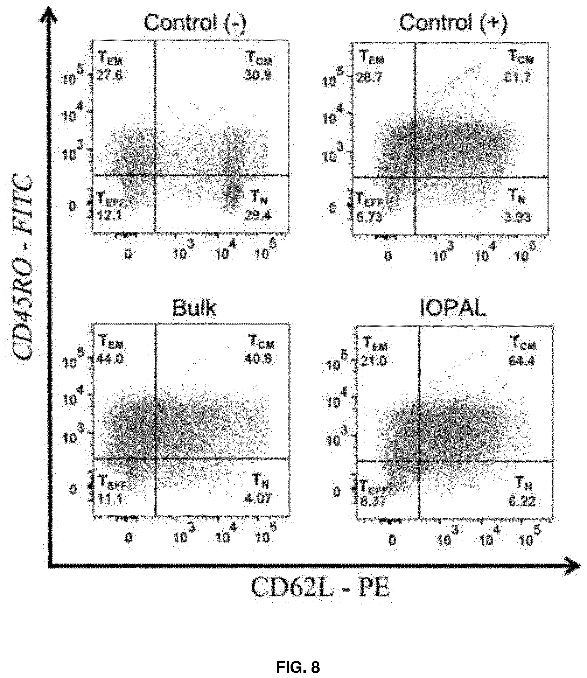

**Fig. 8****.** CD45RO-FITC vs CD62L-PE gate for differentiation of CD4+ T cells in bulk and IOPAL PEG-Hep hydrogels. Percentage of naive (T_{N}), central memory (T_{CM}), effector memory (T_{EM}), and effector (T_{EFF}) CD4+ T cells seeded on bulk and IOPAL PEG-Hep hydrogels (and their controls) on day 5, represented in a CD45RO-FITC Vs CD62L-PE gate. The negative control consists of cells seeded in suspension without Dynabeads, whereas in the positive control, cells are seeded with Dynabeads. When cells are seeded in the hydrogels, they are always stimulated with Dynabeads. This figure shows representative data obtained from 1 donor.

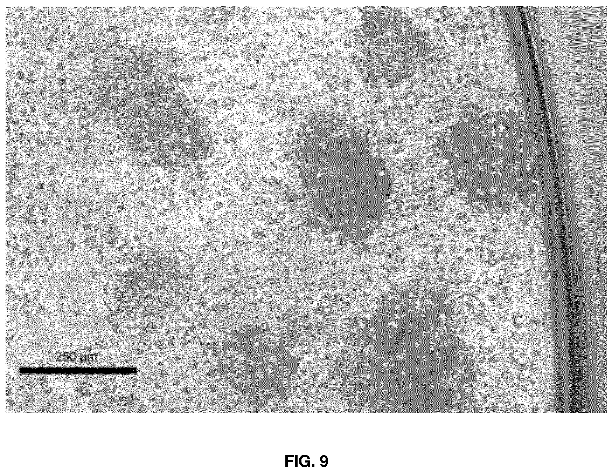

**Fig. 9****.** Microscope image that shows human pancreatic cancer cell (PANC-1) organoids in an IOPAL hydrogel 6 days after seeding obtained in example 4.

### EXAMPLES

### Example 1: Preparation of bulk and IOPAL PEG-Hep hydrogels. Study of the structural properties of the IOPAL PEG-Hep hydrogels.

The IOPAL PEG-Hep hydrogels are hydrogels according to the present invention. However, bulk hydrogels are the hydrogels of the state of the art not having an IOPAL structure. The bulk hydrogels are prepared for comparative purposes.

### 1. Synthesis of bulk and IOPAL PEG-Hep hydrogels

The bulk PEG-Hep hydrogels have been produced as recently reported (Perez Del Río, E. et al. Biomaterials 259, 120313 (2020)). In summary, a Michael-type reaction was used to functionalize the heparin with a maleimide group yielding a Mal-Hep derivative (Baldwin, A. D. & Kiick, K. L. Polym. Chem. 4, 133-143 (2013)). PEG-Hep hydrogels were formed through a maleimide-thiol reaction between the Mal-Hep derivative and a 4-arm thiolated PEG (PEG-SH) in a molar ratio of 1.5:1, in phosphate buffered saline (PBS). This resulted in a covalent crosslink and the consequent hydrogel formation with 3% wt of PEG. In this example though, the volume of each bulk hydrogel used was 30 µL, added to a home-made Teflon template with 5 mm-diameter wells. For the IOPAL PEG-Hep hydrogel formation, 100 µL of a 10% w/v aqueous suspension of PMMA (microParticles GmbH, Germany) 78.3 ± 1.7 µm diameter beads was added in the same home-made well plate template and left for 24 h until solvent evaporation. Thus, the opal was formed. Afterwards, a PEG-Hep hydrogel mixture analogous to the one described above for the bulk PEG-Hep hydrogels (3% wt PEG), was prepared mixing sterile solutions of 4-arm PEG-SH and Mal-Hep in PBS, as explain above. The hydrogel mixture (30 µL) was added on top of the PMMA opal in the template and left to infiltrate and jellify for at least 48 h in the incubator (37ºC). Opal-PEG-Hep hydrogel hybrids were removed from the template after hydrogel formation. Then, the PMMA opal was dissolved by introducing the hybrids in glacial acetic acid (AcOH) for 72 h at 40 ºC and agitation (150 rpm; orbital shaker). After this period, these hydrogels become completely transparent, indicating the successful removal of the PMMA beads. Finally, the resulting IOPAL PEG-Hep hydrogels were washed, sterilized, and incubated until seeding at 37ºC (Figure 1). The resulting IOPAL PEG-Hep hydrogels were washed with PBS to remove the acid. After 1 h of UV sterilization, they were rinsed with the cell culture media (Roswell Park Memorial Institute cell culture medium (RPMI) with 10% fetal bovine serum (FBS) and 1% penicillin-streptomycin) and incubated until seeding at 37ºC.

### 2. Results and Discussion

### 2.1. Structural properties of the IOPAL PEG-Hep hydrogels

Both hydrogels, bulk and IOPAL, were studied by environmental scanning electron microscopy (ESEM) and their pore size ranges were calculated (Figure 2A and B). From the ESEM images, it was calculated the average pore size present in the bulk hydrogels to be 47 µm with a range of 9-204 µm, being these results consistent with the ones previously obtained (Perez Del Río, E. et al. Biomaterials 259, 120313 (2020)). On the other hand, the average pore size present in the IOPAL hydrogels was 77 µm with a range of 24-165 µm, in accordance with the size of the PMMA beads used (78.7 µm ± 1.7 µm).

Additionally, the cell infiltration in the IOPAL hydrogel was assessed by confocal microscopy, by staining primary human CD4+ T cells from healthy donors with carboxyfluorescein diacetate succinimidyl ester (CFSE), an intracellular dye that is only fluorescent in viable cells, used also in the proliferation studies (Figure 2C).

Further physical characterization was performed recurring to the X-ray tomography technique, where it was found a connectivity density for the IOPAL system four times superior when compared with the bulk hydrogel (Figure 3).

### 2.2. Mechanical properties of IOPAL PEG-Hep hydrogels

The mechanical properties of IOPAL PEG-Hep hydrogels were characterized by small-amplitude oscillatory shear (SAOS) rheology (Figure 4). The protocol followed was a cycle of sweeps, namely a strain sweep, and frequency sweep (Zuidema, J. M. et al. J. Biomed. Mater. Res. B. Appl. Biomater. 102, 1063-1073 (2014)). For the strain sweeps (Figure 4A), it was employed a constant frequency of 1.0 Hz and the pressure was swept from 1 Pa to 150 Pa on the fully formed hydrogels. The frequency sweeps (Figure 4B) were performed from 0.01 Hz to 1.0 Hz at a constant strength of 50 Pa.

The mechanical properties of IOPAL PEG-Hep hydrogels were measured in the linear-viscoelastic regime (LVE). The storage modulus (G') obtained for the IOPAL hydrogels was of 0.46 ± 0.01 KPa, which is lower than the one of the bulk hydrogels (0.75 ± 0.08 KPa) (Perez Del Río, E. et al. Biomaterials 259, 120313 (2020)) (Figure 4C). This difference can be explained by the homogeneously larger and more interconnected pores of the IOPAL structure compared to the bulk. These values are comparable with the previously reported values of human secondary lymphoid organs (Hirsch, S. et al. Magn. Reson. Med. 71, 267-277 (2014)).

### Example 2 cell culture experiments: CD4+ T cell viability, expansion, and differentiation using IOPAL PEG-Hep hydrogels.

For the cell culture experiments, primary human CD4+ T cells from healthy adult donors were used due to their natural abundance in comparison with other relevant T cell types such as CD8+ T cells or regulatory T cells, as well as their relevance in the clinics. Bulk and IOPAL PEG-Hep hydrogels were selected to culture CD4+ T cells with Dynabeads. As mentioned above, Dynabeads are magnetic beads coated with anti-CD3 and anti-CD28 that are employed to polyclonally activate T cells. Both hydrogels were used as biomimetic LNs, as they were capable of providing a 3D culture environment, as confirmed by confocal microscopy. The results for the IOPAL hydrogels are shown above (Figure 2C), whereas the ones for the bulk hydrogels were described previously (Perez Del Río, E. etal. Biomaterials 259, 120313 (2020)).

To determine the influence of the increased porosity and interconnectivity given by the IOPAL hydrogels compared to the bulk hydrogels, T cell viability, differentiation, and proliferation were measured by flow cytometry at days 5 or 6, as a standard time points (Lyons, A. B. & Parish, C. R. J. Immunol. Methods 171, 131-137 (1994)).

For T cell viability, a propidium iodide (PI) viability test was performed (Figure 5), which showed more non-viable or apoptotic PI+ cells in Dynabeads activated suspension cultures (control +; 39.9%) than when in bulk (31.8%) or IOPAL (26.1%) hydrogels through flow cytometry. When in suspension without activation, the cells were only 15.5% PI +, after 5 days of culture.

The proliferation assay was performed using CFSE-stained cells analyzed by flow cytometry after 6 days of culture. Then, the expansion index was analyzed, which gives information about the growth of the whole culture, as a ratio between the final and the starting number of cells; the replication index, defined by the fold-expansion of the culture, but only taking into account the activated cells; and the proliferation index related with the average number of divisions that stimulated cells have undergone. The higher this value, the higher the cellular proliferative response provided by the culture environment (Roederer, M. Cytometry. Part A 79, 95-101 (2011)). These three parameters provide information about the cell response to the activation and proliferation stimulus, and also on the total number of cells achieved at the end of the 6 days of proliferation, which is the most important value for cell therapies. Given the donor-to-donor variability, the proliferation results (Figure 6) were normalized in each experiment to the control positive, consisting of CD4+ T cells with Dynabeads in suspension. For that reason, all the positive controls index values are 1.

The median normalized proliferation, expansion, and replication indexes for the bulk hydrogels were 1.03, 1.01, and 1.29 respectively, in accordance with previous results (Perez Del Río, E. et al. Biomaterials 259, 120313 (2020)). The normalized proliferation, expansion, and replication indexes were significantly higher for the IOPAL than the bulk hydrogels, being 1.18, 1.28, and 1.63, respectively. Both hydrogels showed statistically significant increases compared to the positive controls, except for the expansion index in the bulk hydrogel, even though a slight increase of 1% was observed. The most significant improvement was observed for the replication index with the IOPAL hydrogels, showing an improvement of 63% compared to the cells activated in suspension (positive control) and an increase of 34% in comparison with the bulk hydrogel. This indicates that the responding cells that get activated in the hydrogels proliferate more than the activated cells in suspension. Thus, the increase in pore size, homogeneity, and interconnectivity introduced by the inverse opal strategy, contributed to a better overall expansion.

Regarding the differentiation studies, the CD4+ T cell phenotypes were analyzed on day 5 after seeding to obtain information about the subpopulations of naive (TN; CD45RO⁻/CD62L⁺), effector memory (TEM; CD45RO⁺/CD62L⁻), effector (TEM; CD45RO⁻/CD62L⁻), and central memory (TCM; CD45RO⁺/CD62L⁺) T cells, given their clinical importance in ACT (Figure 7). For these analyses, flow cytometry was used (Figure 8).

As mentioned above, there is a donor-to-donor variability that should be taken into account. For this reason, the percentages of CD4+ T cells that express CD45RO and CD62L were also analyzed prior to stimulation with Dynabeads (negative control). The differentiation percentage results with the statistical treatment of the different subpopulations here studied, for 6 different donors, are shown in detailed in Figure 7B-E. The main phenotype obtained was TN with a median value of 45%. This subpopulation was found much lower in the cases where cells were activated, namely positive control, bulk and IOPAL hydrogels, with percentages of 6, 15, and 11%, respectively. For the TEM phenotype, median values rose to 27% for T cells in suspension, 32% for bulk hydrogels, and 19% for IOPAL. Thus, the IOPAL hydrogels did not promote the effector memory phenotype compared to suspension and the bulk hydrogels. In contrast, the TEFF phenotype resulted in lower percentages when cells were activated. A 5, 9, and 7% were obtained for the positive control, bulk and IOPAL hydrogels, whereas the negative control showed a 12% of the total cell population. Remarkably, the median value for the TCM phenotype obtained in CD4+ T cells cultured in the IOPAL hydrogels was of 63%. This percentage is higher than the 43% obtained with the bulk hydrogels and comparable with the 62% obtained in suspension. These results indicate that PEG-Hep hydrogels can be used to better tune the resulting phenotype of T cells only by adding a physical change to their structure, such as the higher pore size, homogeneity, and interconnectivity achieved with the IOPAL strategy. Indeed, the TCM phenotype is of great importance to the clinics due its capacity to mediate an effective and sustained response through proliferating in the SLOs and producing a supply of new TEM cells upon relapse. The last are known to present an immediate, but not sustained, defense response.

### The Materials and Methods used to carry out the examples 1 and 2 are indicated below:

**Materials:** A 10% w/v aqueous suspension of poly(methyl methacrylate) (PMMA) beads was purchased from microParticles GmbH, Germany (78.3 ± 1.7 µm diameter beads), whereas heparin (CAS: 9041-08-1) was purchased from Fisher Scientific (Spain). Fetal bovine serum (FBS), penicillin/streptomycin (P/S), CellTrace CFSE cell proliferation kit, and Dynabeads were supplied by Thermo Fisher Scientific (USA). The CD4+ T cell isolation kit was obtained from Miltenyi Biotec GmbH (Germany). The anti-human CD3 FITC, CD4 PE, CD45RO FITC antibodies and their controls used for flow cytometry were bought from Immunotools GmbH (Germany), whereas CD62L PE and its control were bought from BioLegend (USA). Lymphoprep was acquired from Stemcell Technologies (Canada). PEG-SH (Mn 10,000 g/mol), N-(2-aminoethyl) maleimide trifluoroacetate salt (AEM), 1-hydroxybenzotriazole hydrate (HOBT), N-(3-dimethylamino-propyl)-N-ethylcarbodiimide hydrochloride (EDC·HCl), 2-(N-morpholino) ethanesulfonic acid (MES), Dulbecco's PBS, RPMI-1640 media, and the rest of the products not otherwise specified were obtained from Merck (USA).

### Environmental scanning electron microscopy (SEM): To image the structure of the hydrated bulk and IOPAL hydrogels a FEI Quanta 650F environmental scanning electron microscope (Thermo Fisher Scientific, USA) was chosen.

***X-ray microtomography:*** For these measurements, large IOPAL hydrogels were formed, following the protocol mentioned in example 1 (Synthesis of bulk and IOPAL PEG-Hep hydrogels), except for the amount of PMMA beads and hydrogels precursors mixtures, being respectively 333 µL and 100 µL. For the measurement, a Skyscan 1272 high-resolution micro computed tomography (Bruker, USA) was used to study the 3D structure of the IOPAL hydrogels and analyze the porosity and interconnectivity of the pores. The samples stored in PBS were frozen with liquid nitrogen and then lyophilized before the analysis. The size of the measured samples was 2 mm in height and 1 cm in diameter. The scanning time was 3 h with a minimum resolution of 5 µm, without any filter and with a peak voltage of 40-50kV.

***Rheometry*:** For these measurements, larger IOPAL hydrogels were also formed, using the same amounts indicated above, 333 µL of PMMA beads suspension and 100 µL of hydrogels precursors mixture. The small-amplitude oscillatory shear (SAOS) technique was used to characterize the mechanical properties of the IOPAL and hydrogels at 37°C. Strain sweeps were performed at a constant frequency of 1.0 Hz and a shear stress of 1-50 Pa. The frequency sweeps were performed from 0.01 Hz to 1.0 Hz and a constant shear stress of 50 Pa. The equipment used was a Rheometer HAAKE RheoStress RS600 (Thermo Electron Corporation, USA) with a 10 mm diameter rotor.

*CD4+ T cell culture in bulk and IOPAL PEG-Hep hydrogels:* Buffy coats from healthy adult donors, supplied by "Banc de Sang i Teixits" (Barcelona, Spain) under the approval of the research project by the "Ethics Committee on Animal and Human Experimentation" of the Autonomous University of Barcelona (Nr. 5099) were used. To obtain primary human CD4+T cells, an established protocol (Guasch, J. et al. Nano Lett. 17, 6110-6116 (2017); Guasch, J. et al. Nano Lett. 18, 5899-5904 (2018)) was followed. In summary, it consists of isolating peripheral blood mononuclear cells (PBMCs) by density gradient centrifugation (using Ficoll) and then separating the CD4+ T cells with a commercial CD4+ T cell isolation kit. The purity of the cells was measured by flow cytometry with the antibodies anti-human CD3 FITC, anti-human CD4 PE, and the respective negative controls. Only samples that were > 90% positive for both CD3+ and CD4+ were used (usually CD3+ and CD4+ T cells > 95%). On the cell purification day, the CD4+ T cells were seeded at a concentration of 10⁶ cells/ml in supplemented RPMI medium on the resulting hydrogels (bulk and IOPAL), which were placed in 96-well plates, together with Dynabeads (1:1 ratio). Cells were seeded on the hydrogels, given that their pore size and interconnectivity ensure proper cell infiltration through the structure.

**Confocal microscopy:** The 3D images of a volume of 1.5 mm x 1.5 mm x 0.4 mm were obtained with a Leica TCS SP5 confocal microscope (Leica, Germany) equipped with 10X objectives, on day 5.

### CD4+ T cell viability, proliferation, and differentiation in synthetic PEG-Hep hydrogels:

For cells viability experiments, 0.5 µL of propidium iodide (PI) (1 mg/ml, Merck) was used to stain CD4+ T cells during 3 min at room temperature before flow cytometry measurements with a CytoFLEX LX (Beckman Coulter, USA).

For proliferation analysis, CD4+ T cells were stained with a CFSE cell proliferation kit following the instructions of the manufacturer. At day 6, they were analyzed by flow cytometry with a BD FACSCanto (BD Bioscience, USA) after recovering the cells from the hydrogels through vigorous pipetting. To study the effect of the hydrogels while reducing the variability caused by the different donors the results obtained were normalized to the positive control of each donor, which was assigned a value of 1.

For the differentiation analysis, CD4+ T cells were removed from the hydrogels as explained above and analyzed 5 days after seeding. Anti-human CD45 RO FITC, CD62L PE, and the corresponding negative controls were used to stain the CD4+ T cells for 30 min at 0°C. Then, the cells were washed and examined by flow cytometry with a BD FACSCanto (BD Bioscience, USA).

***Data treatment:*** Cell viability, proliferation, and differentiation data analysis was carried out with the FlowJo software (FlowJo LLC, BD, USA), while data processing was performed with OriginPro (OriginLab Corp. USA), using a non-parametric Mann Whitney U-test to evaluate the statistical significance. For the pore size distributions, the statistical significance was determined by a Kruskal-Wallis test. In the boxplot graphs, the boxes correspond to the interquartile range defined by the 25th and 75th percentiles, the central line is the median, the whiskers show 1 standard deviation, and □ is the average.

### Example 3: Preparation of hydrogels with linear vs 4-arm PEG.

Two different samples were prepared, in which the only difference between them is the number of arms of the thiol-PEG component (2-arm vs 4-arm). To compensate for the 4-arm PEG having twice as thiol reactive groups compared to the linear PEG, the linear PEG was added in a 2-times higher concentration.

**Sample 1:** 6 mM linear PEG-SH and 3.6 mM Heparin-maleimide in 200 µL PBS.

**Sample 2:** 3 mM 4-arm PEG-SH and 3.6 mM Heparin-maleimide in 200 µL PBS.

The gelling of both hydrogels was assessed using an inverted vial test. This simple test consists on turning the vials upside down and establishing whether the starting liquid mixture of components has formed a self-sustainable hydrogel or not.

**Result:** After 24 h at 37 ºC, the inverted vial test showed that Sample 1 continued to exhibit a viscous flow while Sample 2 was self-supportive (i.e. it had formed a hydrogel).

### Example 4: Organoid cell culture in IOPAL PEG-Hep hydrogels.

Two identical IOPAL PEG-Hep hydrogels of 30 µL each were placed in a 96-well plate. Cells from a human pancreatic cancer cell line (PANC-1) (acquired in ATCC (Manassas, VA 20110-2209, USA) were seeded at 5·10⁴ cells/well on top of the hydrogels. The total added volume was 200 µL and cell media consisted of DMEM/F-12 (1:1) supplemented with 1% penicillin/streptavidin, 1 w/v% bovine serum albumin, 17.5 mM glucose, 1x B27, 20 ng/mL basic fibroblast growth factor (bFGF), and 50 ng/mL epidermal growth factor (EGF). The 96-well plate was placed into a cell incubator at 37 ºC and 5% CO₂. Periodical examination was performed using an inverted microscope.

After 4 days of culture, cells already formed large aggregates. These structures increased in size and gained appearance of organoids over cell aggregates with time (Figure 9). The experiment was kept for 14 days.

### Conclusions

In conclusion, IOPAL 3D PEG-Hep hydrogels were synthesized, characterized, and utilized for culturing human cells, especially primary (CD4+) T cells and the pancreatic cancer cell line (PANC-1).

The IOPAL hydrogels provided not only an improvement in cell proliferation when compared to the state-of-the-art methodologies, but also to its bulk form with no IOPAL structure, indicating the importance of the pore size and interconnectivity on T cell activation and proliferation. Moreover, it has been demonstrated the capacity of such hydrogels to influence the phenotype obtained, which is closely related with the clinical outcomes.

Additionally, the IOPAL hydrogels have surprisingly demonstrated an impressive capacity to host organoid formation, especially of solid human tumors, where the current state-of-the-art hydrogels are from murine origin.

The IOPAL strategy here used is expected to better tune a large variety of different hydrogels currently used in the cancer research field, helping to surpass current limitations of ACT, such as producing large amounts of persistent T cells with therapeutic phenotypes, as well as to reduce the animal use in preclinical studies by creating more accurate organoid models.

Additionally, these hydrogels are expected to be compatible with bioreactors working under good manufacturing practice (GMP) conditions, enabling the further culturing and expansion of human cells in large facilities.

## Claims

1. Hydrogel, comprising:
a functionalized PEG multi-arm star polymer covalently combined with heparin wherein the hydrogel is in the form of an inverse opal scaffold.

2. The hydrogel, according to claim 1, wherein PEG is a thiol-functionalized PEG multi-arm star polymer.

3. The hydrogel, according to any of the preceding claims, wherein PEG is a thiol-functionalized PEG 4-arm star polymer.

4. The hydrogel, according to any of the preceding claims, wherein heparin is non-fractionated heparin.

5. The hydrogel, according to any of the preceding claims, wherein heparin is maleimide-functionalized heparin.

6. The hydrogel, according to any of the preceding claims, **characterized in that** it does not comprise cytokines, cell adhesion molecules, or growth factors.

7. The hydrogel, according to any of the preceding claims, wherein the functionalized PEG multi-arm star polymer is between 1.2%wt to 4%wt on the total weight percentage of hydrogel.

8. The hydrogel, according to any of the preceding claims, that has an average pore between 10 µm and 200 µm.

9. A composition for the preparation of the hydrogel of any of the preceding claims comprising the hydrogel of any of the preceding claims and PMMA or polystyrene non-crosslinked polymeric spheres having a diameter between 10 µm and 200 µm.

10. A process for the preparation of the hydrogel in the form of an inverted opal scaffold described in any of the claims 1 to 8, comprising the following steps:
i. adding a solution of non-crosslinked polymeric spheres into a template,
ii. evaporating the solvent comprising the non-crosslinked spheres,
iii. adding an aqueous solution comprising a functionalized PEG multi-arm star polymer and heparin into the template and maintaining the mixture during a time of at least 24 hours to allow the hydrogel components to react with each other and jellify,
iv. dissolving the non-crosslinked polymeric spheres by adding a solution comprising an acid or an organic solvent to the mixture obtained in step iii).

11. The process, according to the preceding claim 10, wherein the solution of step iv. comprises acetic acid.

12. The process, according to any of the preceding claims 10 to 11, wherein the duration of step iv. is between 1 and 5 days.

13. The hydrogel of any of the claims 1 to 8 for use in immunotherapy.

14. Use of the hydrogel of any of the claims 1 to 8 for *in vitro* human cell culture.

15. Use of the hydrogel of any of the claims 1 to 8 for *in vitro* organoid culture.
